# EUROPEAN PATENT SPECIFICATION

(11) **EP 2 621 418 B1**
(45) Date of publication and mention of the grant of the patent: **21.06.2017**
(21) Application number: 11829957.7
(22) Date of filing: 30.09.2011
(51) Int. Cl.: A61F 5/441

(54) **OSTOMY POUCH WITH FILTERING SYSTEM**
OSTOMIEBEUTEL MIT EINEM FILTERSYSTEM
POCHE DE STOMIE À SYSTÈME DE FILTRAGE

(30) Priority: 30.09.2010 US 388331 P; 29.09.2011 US 201113248704
(43) Date of publication of application: 07.08.2013
(73) Proprietor: ConvaTec Technologies Inc., Las Vegas, NV 89169-6754 (US)
(72) Inventor: LESKO, Marc, Skillman NJ 08558 (US); TSAI, Mingliang, Lawrence, Skillman NJ 08558 (US); OBERHOLTZER, Gary, Skillman NJ 08558 (US)
(74) Representative: Viering, Jentschura & Partner mbB Patent- und Rechtsanwälte
(86) International application number: PCT/US2011/054177
(87) International publication number: WO 2012/044910

(56) References cited:
- GB-A- 2 149 306
- GB-A- 2 461 721
- US-A- 5 074 851
- US-A- 5 250 042
- US-A1- 2003 014 023
- US-A1- 2008 004 580
- US-A1- 2009 227 973

## Description

### FIELD OF THE INVENTION

The present invention relates to ostomy appliances and more particularly to ostomy pouches having a filter.

### BACKGROUND OF THE INVENTION

Gas management and odor removal is of very significant concern in ostomy appliances. Despite the advances in various filter designs, there is still a need by ostomates for improved filter performance. Most ostomy filters currently on the market only work for a short time, in many instances less then 1 day. The key complaints have been leakage, clogging which leads to ballooning, insufficient deodorization, and reduced wear time. When some marketed filters stop working there can be leakage of stool outside of the pouch from the filter. Most of filter designs now utilize an oleophobic membrane to protect the filter from leaking in the presence of the enzymes and chemicals from feces. However, commercial ostomy pouches with or without oleophobic membranes have not addressed the clogging issue as effectively as desired by many ostomates. There are also ostomy pouches utilizing multiple membranes to improve both oleophobic and hydrophobic property in the filter, but this design increases the resistance of air flow significantly.

Most commercial ostomy filters are based on radial flow in which the gases flow along the plane of a relatively flat filter rather than directly or axially through the thickness of the filter. Such a radial or planar flow type is used in order to increase the reaction time between gases and the activated carbon. An axial flow type filter is difficult to make because of the difficulties involved in balancing the deodorization efficiency, the air flow requirement, and the profile of the filter. It is, however, desirable to have an axial flow filter that would allow a relatively small filter to be used without sacrificing the deodorization performance. A small filter size is preferred because it allows the filter to be positioned as close as possible to the seam of a pouch so that the face of the filter is not directly in front of the opening of the stoma, thus decreasing the tendency of filter clogging.

US 2003/014023 A1, which is regarded as the closest prior art, discloses a body waste collector which allows the discharge of gases in an ostomy bag over a long time, the ostomy bag formed by sealing together the peripheral edge portions of two films for collecting body wastes, a faceplate coupled to one film of the ostomy bag, a gas-discharging vent provided in the other film of the ostomy bag, and a deodorizing filter. The filter is protected by an intermediate wall comprising fluid inlets allowing the gases to reach the filter. Nevertheless, the intermediate wall has a tendency to stick to the adjacent film, thereby blocking the fluid inlets and producing a ballooning of the waste collector. GB 2 149 306 A discloses an ostomy bag having first and second walls, the former having a stomal orifice and means whereby it can be attached to a wearer, or to an apertured pad worn by the wearer.

Accordingly, there is a need for an improved ostomy appliance which prevents ballooning of the waste collector. Furthermore, there is a need for an axial flow filter in order to minimize the filter profile and to make a small size filter possible.

### SUMMARY OF THE INVENTION

The present invention provides an ostomy appliance as featured in the independent claim. Preferred embodiments of the present invention are described in the dependent claims.

One aspect of the invention in the filter design of the present invention is to improve the clogging resistance by using the newly developed pleated center panel design. This design includes a filter in the front panel of the pouch and a center panel of film that is intended to shield the filter from direct exposure to stool that is expelled from the stoma. The center panel has at least one pleat, and preferably two pleats, that are intentionally formed into the center panel. These pleats allow the gas to travel from the stoma area into the filter area while preventing a majority of the stool from get to and fouling the filter. Without these pleats the center panel has a tendency to block or seal against the front panel of the pouch and not allow gas to get to the filter and thus cause ballooning of the pouch. Optionally, an open cell foam can be added between the face of the filter and the pleats to further reduce any stool that might get past the pleats.

Another aspect of the invention is the incorporation of a filter designed for axial air flow. The pleated design makes possible the use of a small size filter which has a low profile. Both the strip filter and axially flow filter can be combined with the pleated center panel design to improve the clogging resistance.

Another aspect of the invention is a strip filter design, in which the filter is wrapped with an odor barrier film around its perimeter. Such a design allows the filter to maximize its deodorization performance without significantly increasing the air flow resistance.

A test method was devised to mimic actual usage of filtered pouches and their resistance to clogging. Testing was conducted on a specifically designed tilting table test rig. The test rig was designed to hold the test filtered pouches in the vertical position and tilt them to the horizontal position at timed intervals. The tilt table test rig also incorporates a means to inject air into the filtered test pouch to simulate gas produced by the body. The test pouches with filters are also filled with a simulated stool referred to as Feclone, which is commercially sold and mixed to a desired viscosity of 1 part solid to 3 parts water, to challenge the filtering system from a clogging perspective. When the testing is conducted, the filtering system is exposed to the simulated stool and simulated gas at timed intervals of 10 minutes in each position. Pouches are continuously cycled through the vertical and horizontal positions during this tilt table test. During each cycle the test pouches are injected with about 300 cc air to fill each pouch every 10 minutes with a fill time of 5 seconds. When the filter is not clogged, the test pouch deflates normally by releasing the air through the filter system. Testing is continued until all pouches have failed to release air from inside of the pouch, as indicated by the ballooning in the pouch. The criteria for filter failure is when a test filtered pouch fails to deflate and stays fully ballooned for 3 consecutive tilt table cycles. When ballooning occurs, the simulated stool clogs the filter and the filter system can no longer release the air.

Numerous commercial filtering systems were evaluated. Results of various pouch designs, with and without pleats, are summarized in Table 1. Testing showed that the center pleated panel filtering system doubled the life of the filter to around 300 minutes, which is defined by the time that the filter becomes clogged. Testing also showed that the pleated center panel filtering system when used with the foam tripled the life of the filter to around 600 minutes. Results of various pouch designs, with and without pleats, are summarized in Table 1. Clogging resistance was also compared with and without the foam in the filter system.

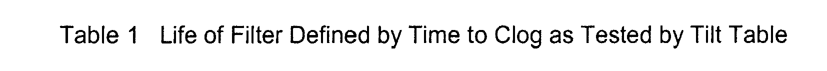

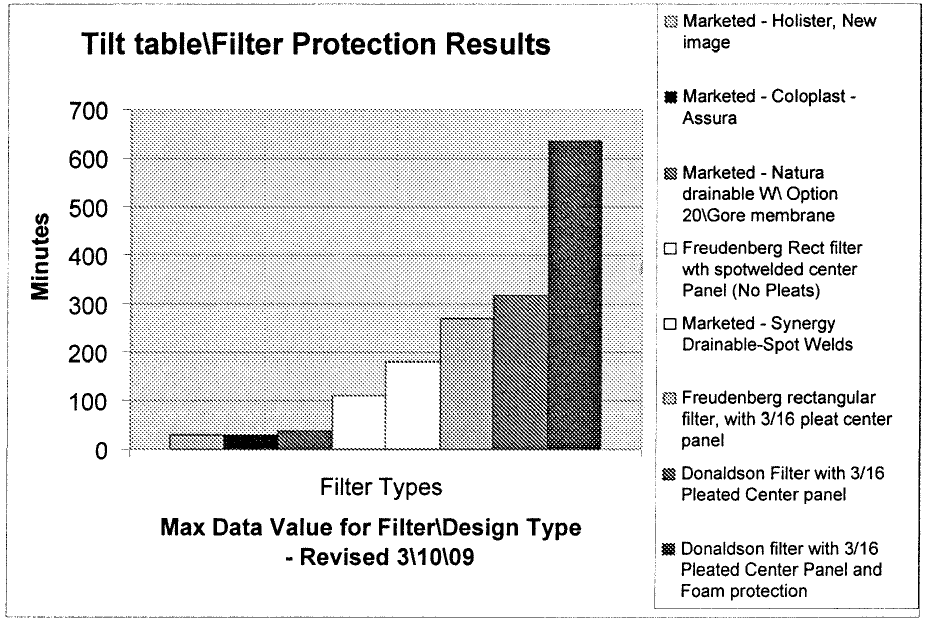

The physical dimension of an axial flow filter versus a radial flow filter is shown in Table 2. Table 3 is a summary of the H₂S deodorization results of the same axial flow filter and the radial flow filter at various relative humidity and with different carrier gases. Table 4 is a graph of the results from Table 3. As can be shown from Table 3 and 4, an axially flow filter outperformed the radial flow filter in the H₂S deodorization even though the activated carbon layer is thinner and the surface area is smaller.

**Table 2 Physical Dimension of an Axial Flow Filter versus a Radial Flow Filter**

| | Radial Flow Filter (Freudenberg Improved Option 20 w/o ePTFE membrane), activated carbon thickness | Axial Flow Filter (Donaldson Nicom 39 with an ePTFE membrane) |
|---|---|---|
| Activated Carbon thickness, mm | 2.2 mm | 1.1 mm |
| Activated Carbon surface area, mm² | 484 | 270 |

**Table 3 H₂S Deodorization Breakthrough Time and Back Pressure**

| | Radial Flow Filter (Freudenberg Improved Option 20 w/o ePTFE membrane), activated carbon thickness | | Axial Flow Filter (Donaldson Nicom 39 with an ePTFE membrane) | |
|---|---|---|---|---|
| %RH and Carrier Gas | H₂S Deodorization Time, minutes | Back Pressure, mbar | H₂S Deodorization Time, minutes | Back Pressure, mbar |
| 0%/ CH₄ and N₂ | 120 - 150 | ∼ 1 | 25-50 | ∼ 10 |
| 0%/Air | 40 | ∼ 1 | N/A | ∼ 10 |
| 5 - 7%/Air, | 170 @ 7%%RH | ∼ 1 | 552 @ 5%RH | ∼ 10 |
| 35%/Air, | 580 | ∼ 1 | 1500 - 2790 | ∼ 10 |
| 35%/CH₄ and N₂ | 320 - 483 | ∼ 1 | 270 | ∼ 10 |
| 35%/Air | 700 - 880 | ∼ 1 | 2353 | ∼ 10 |
| 50%/Air | 960 - 1240 | ∼ 1 | 3090 | ∼ 10 |
| 80%/Air | 1960 | ∼ 1 | 3940 | ∼ 10 |

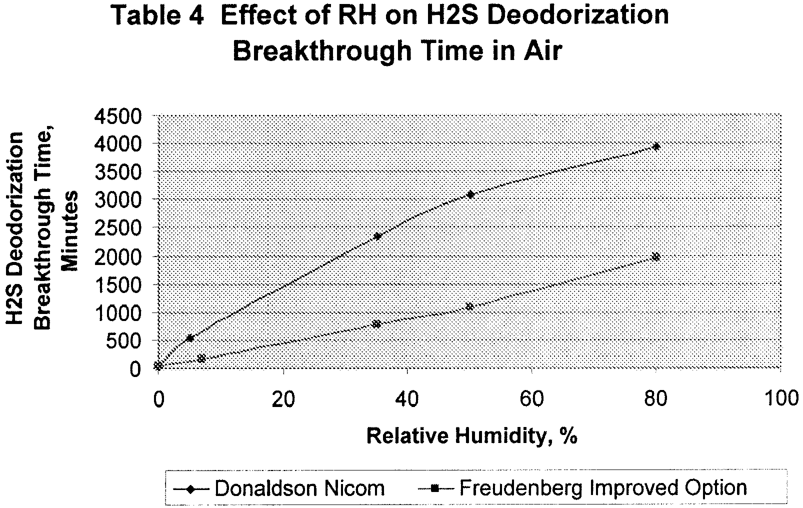

Table 5 is a summary of the H₂S deodorization results of a strip filter, in a rectangular dimension 15 mm x 33 mm, versus a round filter with a diameter of 25.4 mm. Although the total surface area and the activated carbon thickness are about the same, the strip filter had significantly higher deodorization time due to the increased effective flow distance. In this example, the effective flow distance is almost 33% longer, resulting in a longer H₂S deodorization time.

**Table 5 H₂S Deodorization of a Strip Filter versus a Round Filter**

| | Strip Filter (Freudenberg Improved Option 20) | Round Filter (Freudenberg Improved Option 20) |
|---|---|---|
| Activated Carbon thickness, mm | 2.2 mm | 2.2 mm |
| Activated Carbon surface area, mm² | 495 | 484 |
| Effective Flow Distance | 16 mm | 12 mm |
| H₂S Deodorization Time, minutes (0%/ CH₄ and N₂) | 138 | 108 |
| Back Pressure, mbar | 3.1 | 1.0 |

The present invention includes the following:
1. Pleated center panel that improves clogging resistance. An open cell foam can be added to further increase clogging resistance. This pleated design, with and without the foam, can be used with all types of filters, round or strip, radial flow or axial flow. The pleated design, with and without the foam, can be used in either drainable or closed pouch.
2. An axial flow filter with an equivalent or better deodorization time although the activated carbon layer is thinner and the surface area is smaller in an axial flow filter.
3. A strip filter with an equivalent or better deodorization time as a result of the increased effective flow distance, as compared to a round filter which has the about the same thickness and surface area.

### BRIEF DESCRIPTION OF THE DRAWINGS

Fig. 1 is a rear elevational view of an ostomy appliance in accordance with the present invention.
Fig. 2 is a cross-sectional view along line A-A of Fig. 1.
Fig. 3 is a cross-sectional view along line D-D of Fig. 1 (without the stomal flange and filter, for clarity).
Fig. 4 is a front elevational view of the ostomy appliance of Fig. 1 (without the stomal flange, for clarity).

### DETAILED DESCRIPTION OF THE DRAWINGS

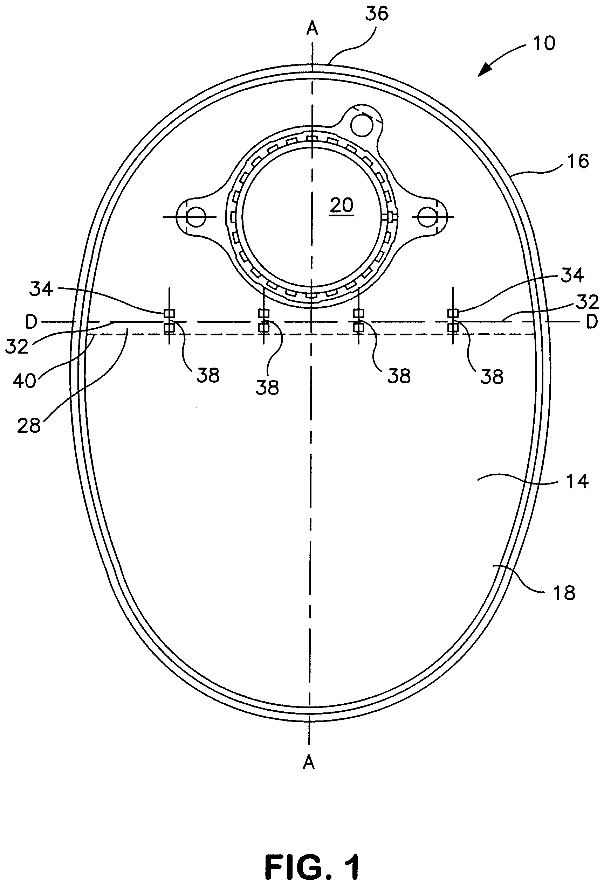

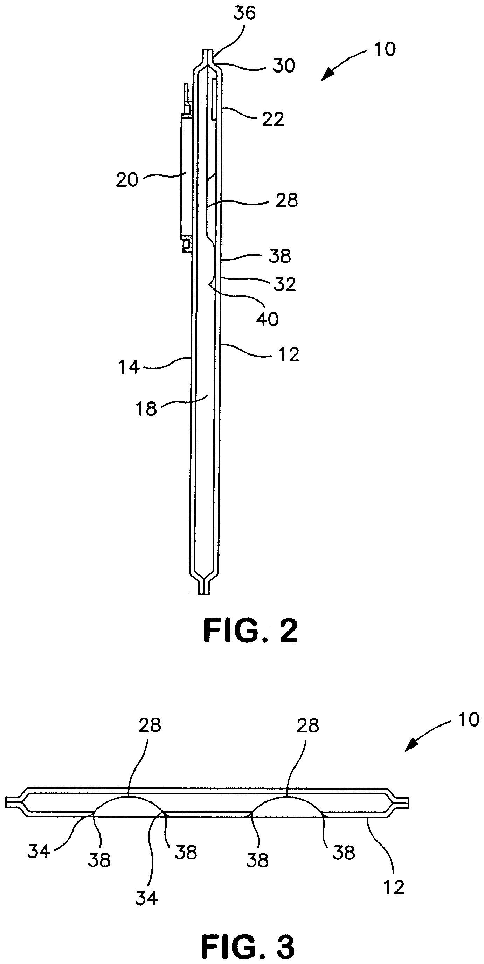

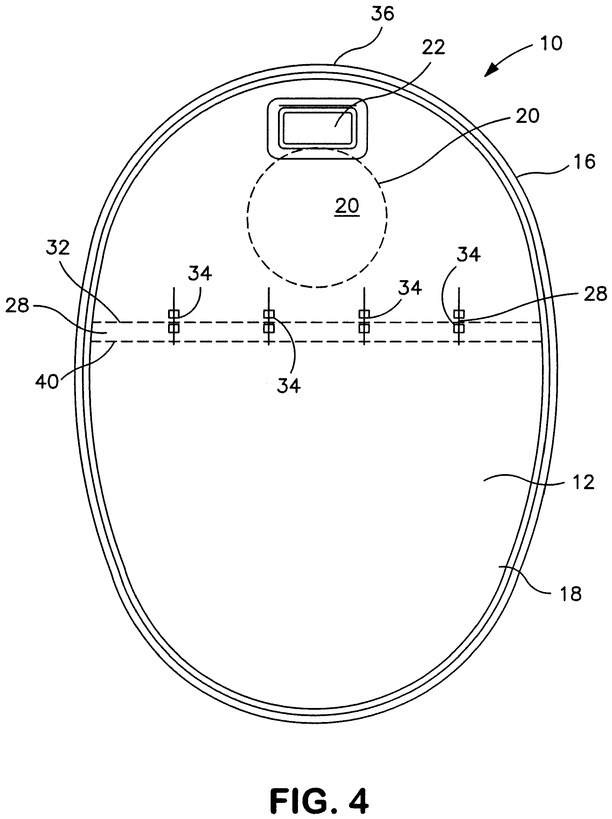

The present invention is an ostomy appliance 10 (Figs. 1-4) having a front panel 12 and rear panel 14 that are sealed together around the peripheries of the panels to form the outer edge 16 of a closed ostomy pouch 18. The rear panel 14 includes a stomal opening 20 through which body waste is excreted when the ostomy pouch is appropriately placed on a body with the stomal opening 20 surrounding the stoma. The ostomy pouch 18 includes a filter assembly 22 attached to the front panel 12 typically by welding. The rear panel 14 includes an opening for permitting and facilitating the passage of the body waste odor deodorized by the filter assembly 22 to the atmosphere.

The ostomy pouch 18 further includes a center pleated panel 28 present between the front panel 12 and rear panel 14. This center pleated panel 28 includes an edge 30 attached to the outer edge 16 of the ostomy pouch 18. The center pleated panel has a partially free edge portion 32 that is attached intermittently 34 to the inner surface of the front panel 12. This center pleated panel 28 is predeterminedly dimensioned and attached to the front panel 12 in a manner to produce extra material in the form of folds, corrugations or pleats 36 within the pouch 18; the pleats permit odorous gas to reach the filter assembly 22 for deodorization while deterring a ballooning of the pouch 18 due to captured gas within the pouch.

The pleated center panel 28 extends partially down from the top 36 of the ostomy pouch so as to at least partially and preferably totally cover and protect the filter assembly 22 from any body waste material entering the pouch 18 through the stomal opening 20. The pleats are formed in part by the spot welds 38 securing the edge portion 32 to the front panel 12. The pleated center panel 28 has a bottom edge 40. The pleated center panel 28 is preferably made of the same standard ostomy pouch material used for the front panel 12 and rear panel 14.

Variations and modifications to the preferred embodiment may be made while falling within the scope of the invention as defined by the claims.

## Claims

1. An ostomy appliance (10) comprising:
two opposing panels (12, 14), comprising a front panel (12) and a rear panel (14), at least partly sealed to one another along their peripheries, said panels (12, 14) forming a pouch (18) for capturing body waste, one of said opposing panels (12, 14) having a stomal opening (20) for positioning around a stoma so that body waste excreted through the stoma is captured in said pouch (18),
said pouch (18) having a filter (22) for deodorization of odorous gas from the body waste and an opening for emitting said deodorized gas to the atmosphere, and a pleated center panel (28) between said two opposing panels (12, 14) at least partly covering said filter (22) for protecting said filter (22) and deterring ballooning of said pouch (18) from gas buildup in said pouch (18),
**characterized in that** the pleated center panel (28) has a partially-free edge portion (32) that is attached intermittently to an inner surface of the front panel (12) in a manner to produce extra material in the form of folds, corrugations or pleats.

2. The ostomy appliance (10) of claim 1, wherein said pleated center panel (28) is suitably folded to maintain openings for facilitating passing of gas to said filter (22).

3. The ostomy appliance (10) of claim 1, further including an open cell foam between said filter (22) and said pleated center panel (28).

4. The ostomy appliance (10) of claim 1, wherein the filter (22) is an axial flow type of filter or strip filter.

## Patentansprüche

1. Eine Stomavorrichtung (10), aufweisend:
zwei einander gegenüberliegende Paneele (12, 14), aufweisend ein Vorderseitenpaneel (12) und ein Rückseitenpaneel (14), welche zumindest teilweise entlang ihrer Umfangsränder miteinander versiegelt sind, wobei die Paneele (12, 14) einen Beutel (18) zum Auffangen von Körperausscheidungen bilden, wobei eines der beiden einander gegenüberliegenden Paneele (12, 14) eine Stomaöffnung (20) hat, welche um ein Stoma herum angeordnet wird, sodass durch das Stoma ausgeschiedene Körperausscheidungen in dem Beutel (18) aufgefangen werden, wobei der Beutel (18) einen Filter (22) zur Desodorierung geruchstragender Gase von den Körperausscheidungen und eine Öffnung hat zum Abgeben der desodorierten Gase an die Umwelt, und ein plissiertes Mittelpaneel (28) zwischen den zwei einander gegenüberliegenden Paneelen (12, 14) zum zumindest teilweisen Abdecken des Filters (22), um den Filter (22) zu schützen und ein Aufblähen des Beutels (18) durch das Ansammeln von Gasen in dem Beutel (18) zu verhindern,
**dadurch gekennzeichnet, dass** das plissierte Mittelpaneel (28) einen teilweise freien Randabschnitt (32) hat, der intermittierend an einer Innenfläche des Vorderseitenpaneels (12) in einer Weise angebracht ist, dass zusätzliches Material in der Form von Falten, Wellen oder Plissees entsteht.

2. Die Stomavorrichtung (10) gemäß Anspruch 1, wobei das plissierte Mittelpaneel (28) geeignet gefaltet ist, sodass Öffnungen erhalten bleiben, um das Durchströmen von Gas zu dem Filter (22) zu erleichtern.

3. Die Stomavorrichtung (10) gemäß Anspruch 1, ferner aufweisend einen offenzelligen Schaumstoff zwischen dem Filter (22) und dem plissierten Mittelpaneel (28).

4. Die Stomavorrichtung (10) gemäß Anspruch 1, wobei der Filter (22) ein Filter vom axial durchströmten Typ oder ein Streifenfilter ist.

## Revendications

1. Appareil de stomie (10) comprenant :
deux panneaux opposés (12, 14), comprenant un panneau avant (12) et un panneau arrière (14), au moins partiellement scellés l'un sur l'autre le long de leurs périphéries, lesdits panneaux (12, 14) formant une poche (18) pour capturer les déchets corporels, l'un desdits panneaux opposés (12, 14) ayant une ouverture de stomie (20) pour se positionner autour d'une stomie de sorte que les déchets corporels excrétés à travers la stomie, sont capturés dans ladite poche (18),
ladite poche (18) ayant un filtre (22) pour la désodorisation des gaz odorants provenant des déchets corporels et une ouverture pour émettre ledit gaz désodorisé à l'atmosphère, et un panneau central plissé (28) entre lesdits deux panneaux opposés (12, 14) recouvrant au moins partiellement ledit filtre (22) pour protéger ledit filtre (22) et empêcher le gonflement de ladite poche (18) provenant de l'accumulation de gaz dans ladite poche (18),
**caractérisé en ce que** le panneau central plissé (28) a une partie de bord partiellement libre (32) qui est fixée de manière intermittente à une surface interne du panneau avant (12) afin de produire un matériau supplémentaire sous la forme de plis, d'ondulations ou de plissements.

2. Appareil de stomie (10) selon la revendication 1, dans lequel ledit panneau central plissé (28) est plié de manière appropriée pour maintenir des ouvertures afin de faciliter le passage du gaz vers ledit filtre (22).

3. Appareil de stomie (10) selon la revendication 1, comprenant en outre une mousse à alvéoles ouvertes entre ledit filtre (22) et ledit panneau central plissé (28).

4. Appareil de stomie (10) selon la revendication 1, dans lequel le filtre (22) est un type de filtre à écoulement axial ou un filtre à bande.
